# EUROPÄISCHE PATENTSCHRIFT

(11) **EP 3 230 443 B1**
(45) Veröffentlichungstag und Bekanntmachung des Hinweises auf die Patenterteilung: **21.07.2021**
(21) Anmeldenummer: 15804109.5
(22) Anmeldetag: 01.12.2015
(51) Int. Cl.: C12N 9/28

(54) **WASCH- UND REINIGUNGSMITTEL MIT EINER KOMBINATION AUS AMYLASE UND PROTEASE**
DETERGENTS AND CLEANING AGENTS COMPRISING A COMBINATION OF AMYLASE AND PROTEASE
AGENT DE LAVAGE ET DE NETTOYAGE AVEC UNE COMBINAISON D'AMYLASE ET DE PROTÉASE

(30) Priorität: 10.12.2014 DE 102014225473
(43) Veröffentlichungstag der Anmeldung: 18.10.2017
(73) Patentinhaber: Henkel AG & Co. KGaA, 40589 Düsseldorf (DE)
(72) Erfinder: ANTIR, Marouane, 1221 JT Hilversum (NL); SCHÜMANN, Sabine, 41470 Neuss (DE); BASTIGKEIT, Thorsten, 42279 Wuppertal (DE); SEILER, Martina, 47228 Duisburg (DE); O'CONNELL, Timothy, 40547 Düsseldorf (DE)
(86) Internationale Anmeldenummer: PCT/EP2015/078166
(87) Internationale Veröffentlichungsnummer: WO 2016/091650

(56) Entgegenhaltungen:
- EP-A1- 2 100 948
- EP-A1- 2 679 679
- WO-A1-99/63040
- WO-A1-2015/044448
- WO-A2-2011/100410
- DATABASE Geneseq [Online] 8. Mai 2014 (2014-05-08), "Alkaline protease variant R99E, SEQ ID 3.", XP002753532, gefunden im EBI accession no. GSP:BBD44632 Database accession no. BBD44632
- None

### SEQUENCE LISTING

<110> Henkel AG & Co. KGaA
<120> Wasch- und Reinigungsmittel mit einer Kombination aus Amylase und Protease
<130> PT032921PCT
<150> 102014225473.6
   <151> 2014-12-10
<160> 4
<170> PatentIn version 3.5
<210> 1
   <211> 485
   <212> PRT
   <213> Artificial
<220>
   <223> Hybridprotein
<400> 1
   His His Asn Gly Thr Asn Gly Thr Met Met Gln Tyr Phe Glu Trp Tyr
   1               5                   10                  15
   Leu Pro Asn Asp Gly Asn His Trp Asn Arg Leu Asn Ser Asp Ala Ser
               20                  25                  30
   Asn Leu Lys Ser Lys Gly Ile Thr Ala Val Trp Ile Pro Pro Ala Trp
           35                  40                  45
   Lys Gly Ala Ser Gln Asn Asp Val Gly Tyr Gly Ala Tyr Asp Leu Tyr
       50                  55                  60
   Asp Leu Gly Glu Phe Asn Gln Lys Gly Thr Val Arg Thr Lys Tyr Gly
   65                  70                  75                  80
   Thr Arg Ser Gln Leu Gln Ala Ala Val Thr Ser Leu Lys Asn Asn Gly
                   85                  90                  95
   Ile Gln Val Tyr Gly Asp Val Val Met Asn His Lys Gly Gly Ala Asp
               100                 105                 110
   Ala Thr Glu Met Val Arg Ala Val Glu Val Asn Pro Asn Asn Arg Asn
           115                 120                 125
   Gln Glu Val Thr Gly Glu Tyr Thr Ile Glu Ala Trp Thr Arg Phe Asp
       130                 135                 140
   Phe Pro Gly Arg Gly Asn Thr His Ser Ser Phe Lys Trp Arg Trp Tyr
   145 150 155 160
   His Phe Asp Gly Val Asp Trp Asp Gln Ser Arg Arg Leu Asn Asn Arg
   165 170 175
   Ile Tyr Lys Phe Arg Gly His Gly Lys Ala Trp Asp Trp Glu Val Asp
   180 185 190
   Thr Glu Asn Gly Asn Tyr Asp Tyr Leu Met Tyr Ala Asp Ile Asp Met
   195 200 205
   Asp His Pro Glu Val Val Asn Glu Leu Arg Asn Trp Gly Val Trp Tyr
   210 215 220
   Thr Asn Thr Leu Gly Leu Asp Gly Phe Arg Ile Asp Ala Val Lys His
   225 230 235 240
   Ile Lys Tyr Ser Phe Thr Arg Asp Trp Ile Asn His Val Arg Ser Ala
   245 250 255
   Thr Gly Lys Asn Met Phe Ala Val Ala Glu Phe Trp Lys Asn Asp Leu
   260 265 270
   Gly Ala Ile Glu Asn Tyr Leu Gln Lys Thr Asn Trp Asn His Ser Val
   275 280 285
   Phe Asp Val Pro Leu His Tyr Asn Leu Tyr Asn Ala Ser Lys Ser Gly
   290 295 300
   Gly Asn Tyr Asp Met Arg Asn Ile Phe Asn Gly Thr Val Val Gln Arg
   305 310 315 320
   His Pro Ser His Ala Val Thr Phe Val Asp Asn His Asp Ser Gln Pro
   325 330 335
   Glu Glu Ala Leu Glu Ser Phe Val Glu Glu Trp Phe Lys Pro Leu Ala
   340 345 350
   Tyr Ala Leu Thr Leu Thr Arg Glu Gln Gly Tyr Pro Ser Val Phe Tyr
   355 360 365
   Gly Asp Tyr Tyr Gly Ile Pro Thr His Gly Val Pro Ala Met Arg Ser
   370 375 380
   Lys Ile Asp Pro Ile Leu Glu Ala Arg Gln Lys Tyr Ala Tyr Gly Pro
   385 390 395 400
   Gln His Asp Tyr Ile Asp His Pro Asp Val Ile Gly Trp Thr Arg Glu
                   405                 410                 415
   Gly Asp Ser Ser Ala Ala Lys Ser Gly Leu Ala Ala Leu Ile Thr Asp
               420                 425                 430
   Gly Pro Gly Gly Ser Lys Arg Met Tyr Ala Gly Leu Lys Asn Ala Gly
           435                 440                 445
   Glu Thr Trp Tyr Asp Ile Thr Gly Asn Arg Ser Asp Thr Val Lys Ile
       450                 455                 460
   Gly Ser Asp Gly Trp Gly Glu Phe His Val Asn Asp Gly Ser Val Ser
   465                 470                 475                 480
   Ile Tyr Val Gln Lys
                   485
<210> 2
   <211> 269
   <212> PRT
   <213> Bacillus lentus
<400> 2
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 3
   <211> 275
   <212> PRT
   <213> Bacillus amyloliquefaciens
<400> 3
<210> 4
   <211> 269
   <212> PRT
   <213> Artificial
<220>
   <223> BLAP R99E
<400> 4
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Glu Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265

## Beschreibung

Die Erfindung betrifft ein Wasch und Reinigungsmittel enthaltend eine Enzymkombination aus einer Amylase und einer Protease, deren Aminosäuresequenzen insbesondere im Hinblick auf den Einsatz in Wasch- und Reinigungsmitteln verändert wurden und alle hinreichend ähnlichen Amylasen und Proteasen mit einer entsprechenden Veränderung. Die Erfindung betrifft ferner Verfahren und Verwendungen dieser Wasch- und Reinigungsmittel.

Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisin-like Proteases" von R. Siezen, Seite 75-95 in "Subtilisin enzymes", herausgegeben von R. Bott und C. Betzel, New York, 1996. Subtilasen werden natürlicherweise von Mikroorganismen gebildet. Hierunter sind insbesondere die von Bacillus-Spezies gebildeten und sezernierten Subtilisine als bedeutendste Gruppe innerhalb der Subtilasen zu erwähnen.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die Protease aus Bacillus lentus, insbesondere aus Bacillus lentus DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so beispielsweise für den Einsatz in Wasch-und Reinigungsmitteln optimiert. Dazu gehören Punktmutagenese, Deletions- oder Insertionsmutagenese oder Fusion mit anderen Proteinen oder Proteinteilen. So sind für die meisten aus dem Stand der Technik bekannten Proteasen entsprechend optimierte Varianten bekannt.

Weitere in Wasch und Reinigungsmitteln einsetzbare Enzyme sind Amylasen. Für Amylasen können synonyme Begriffe verwendet werden, beispielsweise 1,4-alpha-D-Glucan-Glucanohydrolase oder Glycogenase. Erfindungsgemäß bevorzugte Amylasen sind α-Amylasen. Entscheidend dafür, ob ein Enzym eine α-Amylase im Sinne der Erfindung ist, ist deren Fähigkeit zur Hydrolyse von α-(1-4)-Glykosidbindungen in der Amylose der Stärke.

Im Stand der Technik bekannte Amylasen sowie insbesondere auch deren für den Einsatz in Wasch- oder Reinigungsmitteln verbesserte Weiterentwicklungen sind im Folgenden geschildert. Die α-Amylase aus Bacillus licheniformis (erhältlich als Termamyl® von Novozymes oder Purastar®ST von dem Unternehmen Danisco/Genencor) und deren Weiterentwicklungsprodukte Duramyl® und Termamyl®ultra (Novozymes), Purastar®OxAm (Danisco/Genencor) und Keistase® (Daiwa Seiko Inc.), sowie die α-Amylasen aus Bacillus amyloliquefaciens oder aus Bacillus stearothermophilus sind geeignete Amylasen. Die α-Amylase von Bacillus amyloliquefaciens wird von dem Unternehmen Novozymes unter dem Namen BAN® vertrieben, und abgeleitete Varianten von der α-Amylase aus Bacillus stearothermophilus unter den Namen BSG® und Novamyl®, ebenfalls von dem Unternehmen Novozymes. Weitere bekannte Amylasen sind die α-Amylase aus Bacillus sp. A 7-7 (DSM 12368) und die Cyclodextrin-Glucanotransferase (CGTase) aus Bacillus agaradherens (DSM 9948) sowie die unter den Handelsnamen Fungamyl® von dem Unternehmen Novozymes erhältlichen Weiterentwicklungen der α-Amylase aus Aspergillus niger und A. oryzae. Weitere vorteilhaft in Wasch- und Reinigungsmitteln einsetzbare Handelsprodukte sind beispielsweise die Amylase-LT® und Stainzyme® oder Stainzyme ultra® bzw. Stainzyme plus®, letztere ebenfalls von dem Unternehmen Novozymes. Auch durch Punktmutationen erhältliche Varianten dieser Enzyme können in Wasch- und Reinigungsmitteln eingesetzt werden. Besonders bevorzugte Amylasen sind offenbart in den internationalen Offenlegungsschriften WO 00/60060, WO 03/002711, WO 03/054177 und WO07/079938.

Auch aus WO 2011/100410, EP 2679679, WO 2015/044448, EP 2100948 und WO 99/63040 sind für Wasch- und Reinigungsmittel geeignete Amylasen und Proteasen bekannt. Überraschenderweise wurde nun festgestellt, dass eine erfindungsgemäße α-Amylase die Stabilität und die Leistung einer Protease in einem Wasch- und Reinigungsmittel verbessert.

Gegenstand der vorliegenden Erfindung ist daher ein Wasch und-Reinigungsmittel enthaltend eine Kombination aus einer α-Amylase und einer Protease, wobei die α-Amylase dadurch gekennzeichnet ist, dass sie zu der in SEQ ID NO.1 angegeben Sequenz über deren Gesamtlänge zu mindestens 89% und zunehmend bevorzugt zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% und bis zu 100% identisch ist und in der Zählung gemäß SEQ ID NO. 1 an einer oder mehreren der Positionen 180, 181, 182, 183 und 184 eine Deletion aufweist, und wobei die Protease dadurch gekennzeichnet ist, dass sie eine Aminosäuresequenz aufweist, die zu der in SEQ ID NO. 2 angegebenen Aminosäuresequenz über deren Gesamtlänge zu mindestens 80% und zunehmend bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist, wobei die α-Amylase die Stabilität und die Leistung der Protease in dem Wasch- und Reinigungsmittel verbessert.

Besonders bevorzugt ist eine Deletion von zwei Positionen ausgewählt aus 180+181, 181+182, 182+183 und 183+184, ganz besonders bevorzugt sind Deletionen an den Positionen 183+184 in der Zählung gemäß SEQ ID NO. 1, insbesondere bevorzugt die Deletionen H183* + G184*. Vorzugsweise weist die erfindungsgemäße α-Amylase in der Zählung gemäß SEQ ID NO. 1 weiterhin eine Aminosäuresubstitution an einer oder mehreren der Positionen 405, 421, 422 und 428 auf. Besonders bevorzugt sind die Substitutionen I405L; A421H, A422P und A428T.

In einer besonders bevorzugten Ausführungsform weist die erfindungsgemäße α-Amylase in der Zählung gemäß SEQ ID NO. 1 die Deletionen H183* + G184* und zusätzlich die Substitutionen I405L, A421H, A422P und A428T auf.

Die erfindungsgemäße Protease umfasst eine Aminosäuresequenz, die zu der in SEQ ID NO. 2 angegebenen Aminosäuresequenz über deren Gesamtlänge zu mindestens 80% und zunehmend bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, oder zu 100% identisch ist.

Bevorzugt ist eine Protease, die eine Aminosäuresequenz, die zu der in SEQ ID NO. 2 angegebenen Aminosäuresequenz über deren Gesamtlänge zu mindestens 80% und zunehmend bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99% identisch ist und an Position 99 die Aminosäure Glutaminsäure (E) aufweist.

Besonders bevorzugt ist eine Protease gemäß SEQ ID NO.4.

Aminosäurepositionen, die im Rahmen der vorliegenden Erfindung mit der Formulierung "Zählung gemäß SEQ ID NO. 1" angegeben werden, verstehen sich folgendermaßen: Die weiteren Aminosäurepositionen werden durch ein Alignment der Aminosäuresequenz einer erfindungsgemäßen Amylase oder Protease mit der Aminosäuresequenz, wie sie in SEQ ID NO. 1 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz eines erfindungsgemäßen Proteins eine höhere Zahl von Aminosäureresten umfasst als die Amylase oder Protease in SEQ ID NO. 1. Ausgehend von den genannten Positionen in der Aminosäuresequenz sind die Veränderungspositionen in einer erfindungsgemäßen Amylase oder Protease diejenigen, die eben diesen Positionen in einem Alignement zugeordnet sind.

Die erfindungsgemäße Kombination einer erfindungsgemäßen α-Amylase mit einer Protease in Wasch- und Reinigungsmitteln bewirkt eine verbesserte Reinigungsleistung des Wasch- und Reinigungsmittels an mindestens einer Protease-sensitiven Anschmutzung. Erfindungsgemäße α-Amylasen haben eine leistungsverbessernde Wirkung auf die ebenfalls im Wasch- und Reinigungsmittel enthaltene Protease und ermöglichen folglich unter anderem durch ihre Proteasestabilisierende Wirkung eine verbesserte Entfernung von mindestens einer, bevorzugt von mehreren Protease-sensitiven Anschmutzungen auf Textilien und/oder harten Oberflächen, beispielsweise Geschirr. Besonders vorteilhafte Reinigungsleistungen zeigen bevorzugte Ausführungsformen erfindungsgemäßer Wasch-und Reinigungsmittel an Blut-haltigen Anschmutzungen, beispielsweise an der Anschmutzung Blood/Milk/Ink: Produkt Nr. CFT C-05 erhältlich von CFT (Center For Testmaterials) B.V. Vlaardingen, Niederlande.

Durch bevorzugte Ausführungsformen der vorliegenden Erfindung werden Wasch-und Reinigungsmittel mit verbesserter Reinigungsleistung gezielt im Hinblick auf Protease-sensitive Anschmutzungen hergestellt.

Bevorzugte Ausführungsformen erfindungsgemäßer Amylase und Protease Kombinationen erzielen solche vorteilhaften Reinigungsleistungen auch schon bei niedrigen Temperaturen, insbesondere in den Temperaturbereichen zwischen 10°C und 60°C, bevorzugt zwischen 15°C und 50°C und besonders bevorzugt zwischen 20°C und 40°C. Weitere bevorzugte Ausführungsformen erfindungsgemäßer Amylase und Protease Kombinationen erzielen solche vorteilhaften Reinigungsleistungen in einem weiten Temperaturbereich, beispielsweise zwischen 15°C und 90°C, vorzugsweise zwischen 20°C und 60°C.

Unter Reinigungsleistung wird im Rahmen der Erfindung die Aufhellungsleistung an einer oder mehreren Anschmutzungen, insbesondere auf Wäsche oder Geschirr, verstanden. Im Rahmen der Erfindung weisen sowohl das Wasch- oder Reinigungsmittel, welches die Kombination aus Amylase und Protease umfasst beziehungsweise die durch dieses Mittel gebildete Wasch- oder Reinigungsflotte, als auch die Protease und Amylase selbst eine jeweilige Reinigungsleistung auf. Die Reinigungsleistung der Enzyme trägt somit zur Reinigungsleistung des Mittels beziehungsweise der durch das Mittel gebildeten Wasch- oder Reinigungsflotte bei. Die Reinigungsleistung wird bevorzugt ermittelt wie weiter unten angegeben.

Für die vorliegende Erfindung wird vornehmlich die Reinigungsleistung der Protease beleuchtet, wobei die synergistische Wirkung der erfindungsgemäßen α-Amylase indirekt durch die verbesserte Reinigungsleistung der Protease auf Protease-haltigen Anschmutzungen gezeigt wird.

Eine Protease weist eine proteolytische Aktivität auf, das heißt, sie ist zur Hydrolyse von Peptidbindungen eines Polypeptids oder Proteins befähigt, insbesondere in einem Wasch- oder Reinigungsmittel. Eine Protease ist daher ein Enzym, welches die Hydrolyse von Peptidbindungen katalysiert und dadurch in der Lage ist, Peptide oder Proteine zu spalten. Bei den erfindungsgemäßen Amylasen und Proteasen handelt es sich vorzugsweise um die reife (mature) Amylase/Protease, d.h. um das katalytisch aktive Molekül ohne Signal- und/oder Propeptid(e). Soweit nicht anders angegeben beziehen sich auch die angegebenen Sequenzen auf jeweils reife Enzyme.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, daß ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen beziehungsweise Algorithmen basieren. In der vorliegenden Patentanmeldung wurden alle Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern erstellt, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Die Reinigungsleistung wird in einem Waschsystem bestimmt, das ein Waschmittel in einer Dosierung zwischen 3,5 und 6,5 Gramm pro Liter Waschflotte sowie die Protease und die Amylase enthält. Die zu vergleichenden Amylasen werden konzentrationsgleich (bezogen auf aktives Protein) eingesetzt. Die Reinigungsleistung der Protease wird gegenüber einer Blut-Anschmutzung durch Messung des Weißheitsgrades der gewaschenen Textilien bestimmt. Der Waschvorgang erfolgt für 70 Minuten bei einer Temperatur von 40°C, wobei das Wasser eine Wasserhärte zwischen 13,5 und 16,5° (deutsche Härte) aufweist.

Die Konzentration der Protease in dem für dieses Waschsystem bestimmten Waschmittel beträgt von 0,001-0,1 Gew.-%, vorzugsweise von 0,01 bis 0,06 Gew.-%, bezogen auf aktives Protein. Die Konzentration der Amylase in dem für dieses Waschsystem bestimmten Waschmittel beträgt von 0,001-0,15 Gew.-%, vorzugsweise von 0,005 bis 0,012 Gew.-%, bezogen auf aktives Protein.

Ein bevorzugtes flüssiges Waschmittel für ein solches Waschsystem ist wie folgt zusammengesetzt (alle Angaben in Gewichts-Prozent): 0,3-0,5% Xanthan, 0,2-0,4% Anti-Schaummittel, 6-7% Glycerin, 0,3-0,5% Ethanol, 4-7% FAEOS (Fettalkoholethersulfat), 5-15% nichtionische Tenside, 5-15% anionische Tenside (LAS), 1% Borsäure, 1-4% Natriumcitrat (Dihydrat), 2-4% Soda, 2-6% Kokosnuss-Fettsäuren, 0,5-2,5% HEDP (1-Hydroxyethan-(1,1-di-phosphonsäure)), 0-0,4% PVP (Polyvinylpyrrolidon), 0-0,15% optischer Aufheller, 0-0,001% Farbstoff, Rest demineralisiertes Wasser. Bevorzugt beträgt die Dosierung des flüssigen Waschmittels zwischen 3,5 und 6,0 Gramm pro Liter Waschflotte, beispielsweise 4,7, 4,9 oder 5,9 Gramm pro Liter Waschflotte. Bevorzugt wird gewaschen in einem pH-Wertebereich zwischen pH 8 und pH 10,5, bevorzugt zwischen pH 8 und pH 9.

Ein bevorzugtes pulverförmiges Waschmittel für ein solches Waschsystem ist wie folgt zusammengesetzt (alle Angaben in Gewichts-Prozent): 10% lineares Alkylbenzolsulfonat (Natrium-Salz), 1,5% C12-C18-Fettalkoholsulfat (Natrium-Salz), 2,0% C12-C18-Fettalkohol mit 7 EO, 20% Natriumcarbonat, 6,5% Natriumhydrogencarbonat, 4,0% amorphes Natriumdisilikat, 17% Natriumcarbonat-peroxohydrat, 4,0% TAED, 3,0% Polyacrylat, 1,0% Carboxymethylcellulose, 1,0% Phosphonat, 27% Natriumsulfat, Rest: Schauminhibitoren, optischer Aufheller, Duftstoffe. Bevorzugt beträgt die Dosierung des pulverförmigen Waschmittels zwischen 4,5 und 7,0 Gramm pro Liter Waschflotte, beispielsweise und besonders bevorzugt 4,7 Gramm pro Liter Waschflotte, oder 5,5, 5,9 oder 6,7 Gramm pro Liter Waschflotte. Bevorzugt wird gewaschen in einem pH-Wertebereich zwischen pH 9 und pH 11.

Der Weißheitsgrad, d.h. die Aufhellung der Anschmutzungen, als Maß für die Reinigungsleistung wird bevorzugt mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration kann diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913) erfolgen.

Zusätzlich zu den vorstehend erläuterten Aminosäureveränderungen können erfindungsgemäße Amylasen und Proteasen weitere Aminosäureveränderungen, insbesondere AminosäureSubstitutionen, -Insertionen oder -Deletionen, aufweisen. Solche Amylasen und Proteasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Amylasen und Proteasen oder anderer Polypeptide genutzt werden.

Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität der Amylase oder Protease erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Amylase oder Protease, zum Beispiel hinsichtlich ihrer Stabilität gegenüber Tensiden und/oder Bleichmitteln und/oder anderen Komponenten, kann daher im Rahmen der Erfindung zusätzlich weiterentwickelt sein. Für die Beschreibung von Substitutionen, die genau eine Aminosäureposition betreffen (Aminosäureaustausche), wird folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt. Beispielsweise beschreibt A95G die Substitution von Alanin an Position 95 durch Glycin, A95AG die Insertion von Glycin nach der Aminosäure Alanin an Position 95 und A95* die Deletion von Alanin an Position 95. Diese Nomenklatur ist dem Fachmann auf dem Gebiet der Enzymtechnologie bekannt.

Ein weiterer Gegenstand der vorliegenden Erfindung ist daher ein Wasch und-Reinigungsmittel enthaltend eine Kombination aus einer α-Amylase und einer Protease, wobei die α-Amylase dadurch gekennzeichnet ist, dass sie aus einer erfindungsgemäßen Amylase als Ausgangsmolekül durch ein- oder mehrfache konservative Aminosäuresubstitution erhältlich ist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Ein weiterer Gegenstand der vorliegenden Erfindung ist daher ein Wasch und-Reinigungsmittel enthaltend eine Kombination aus einer α-Amylase und einer Protease, wobei die α-Amylase dadurch gekennzeichnet ist, dass sie aus einer erfindungsgemäßen Amylase als Ausgangsmolekül erhältlich ist durch Fragmentierung, Deletions-, Insertions- oder Substitutionsmutagenese und eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 475, 480, 482, 484 oder 485 zusammenhängenden Aminosäuren mit dem Ausgangsmolekül übereinstimmt.

So ist es beispielsweise möglich, an den Termini oder in den Loops des Enzyms einzelne Aminosäuren zu deletieren, ohne daß dadurch die proteolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms. Auch Substitutionen können vorteilhafte Wirkungen zeigen. Sowohl einzelne wie auch mehrere zusammenhängende Aminosäuren können gegen andere Aminosäuren ausgetauscht werden.

Eine erfindungsgemäße Amylase kann zusätzlich stabilisiert sein, insbesondere durch eine oder mehrere Mutationen, beispielsweise Substitutionen, oder durch Kopplung an ein Polymer. Denn eine Erhöhung der Stabilität bei der Lagerung und/oder während des Einsatzes, beispielsweise beim Waschprozess, führt dazu, daß die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Hierfür geeignete Sequenzveränderungen sind aus dem Stand der Technik bekannt.

Weitere Möglichkeiten der Stabilisierung sind z. B.:
- Veränderung der Bindung von Metallionen, insbesondere der Calcium-Bindungsstellen, beispielsweise durch Austauschen von einer oder mehreren der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein weiterer Gegenstand der Erfindung ist eine Amylase wie vorstehend beschrieben, die dadurch gekennzeichnet ist, daß sie mindestens eine chemische Modifikation aufweist. Eine Amylase mit einer solchen Veränderung wird als Derivat bezeichnet, d.h. die Amylase ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können beispielsweise in vivo durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch in vitro durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern.

Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, daß es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden.

Zu dem Erfindungsgegenstand zählen alle denkbaren Wasch- oder Reinigungsmittelarten, sowohl Konzentrate als auch unverdünnt anzuwendende Mittel, zum Einsatz im kommerziellen Maßstab, in der Waschmaschine oder bei der Handwäsche beziehungsweise -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel können weitere Enzyme enthalten. Als weitere Enzyme einsetzbar sind beispielsweise Lipasen oder Cutinasen, insbesondere wegen ihrer Triglycerid-spaltenden Aktivitäten, aber auch, um aus geeigneten Vorstufen in situ Persäuren zu erzeugen. Hierzu gehören beispielsweise die ursprünglich aus Humicola lanuginosa (Thermomyces lanuginosus) erhältlichen, beziehungsweise weiterentwickelten Lipasen, insbesondere solche mit dem Aminosäureaustausch D96L. Hierzu gehören beispielsweise die ursprünglich aus Humicola lanuginosa (Thermomyces lanuginosus) erhältlichen, beziehungsweise weiterentwickelten Lipasen, insbesondere solche mit einem oder mehreren der folgenden Aminosäureaustausche ausgehend von der genannten Lipase in den Positionen D96L, T213R und/oder N233R., besonders bevorzugt T213R und N233R. Des Weiteren sind beispielsweise die Cutinasen einsetzbar, die ursprünglich aus Fusarium solani pisi und Humicola insolens isoliert worden sind. Einsetzbar sind weiterhin Lipasen, beziehungsweise Cutinasen, deren Ausgangsenzyme ursprünglich aus Pseudomonas mendocina und Fusarium solanii isoliert worden sind.

Die erfindungsgemäßen Mittel können auch Cellulasen oder Hemicellulasen wie Mannanasen, Xanthanlyasen, Pektinlyasen (=Pektinasen), Pektinesterasen, Pektatlyasen, Xyloglucanasen (=Xylanasen), Pullulanasen oder β-Glucanasen enthalten.

Zur Erhöhung der bleichenden Wirkung können erfindungsgemäß Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Als weitere Enzyme einsetzbar sind auch Amylasen, wobei alle oben bereits beschriebenen Amylasen geeignet sind.

Die Wasch- oder Reinigungsmittel können neben den zuvor beschriebenen Inhaltsstoffen reinigungsaktive Substanzen enthalten, wobei Substanzen aus der Gruppe der Tenside, Gerüststoffe, Polymere, Glaskorrosionsinhibitoren, Korrosionsinhibitoren, Duftstoffe und Parfümträger bevorzugt werden. Diese bevorzugten Inhaltsstoffe werden in der Folge näher beschrieben.

Ein bevorzugter Bestandteil der erfindungsgemäßen Wasch- oder Reinigungsmittel sind die nichtionischen Tenside, wobei nichtionische Tenside der allgemeinen Formel R¹-CH(OH)CH₂O-(AO)_{w}-(A'O)ₓ-(A"O)_{y}-(A"'O)_{z}-R², in der
- R¹ für einen geradkettigen oder verzweigten, gesättigten oder ein- bzw. mehrfach ungesättigten C₆₋₂₄-Alkyl- oder -Alkenylrest steht;
- R² für einen linearen oder verzweigten Kohlenwasserstoffrest mit 2 bis 26 Kohlenstoffatomen steht;
- A, A', A" und A"' unabhängig voneinander für einen Rest aus der Gruppe -CH₂CH₂, -CH₂CH₂-CH₂, -CH₂-CH(CH₃), -CH₂-CH₂-CH₂-CH₂, -CH₂-CH(CH₃)-CH₂-, -CH₂-CH(CH₂-CH₃) stehen,
- w, x, y und z für Werte zwischen 0,5 und 120 stehen, wobei x, y und/oder z auch 0 sein können
bevorzugt sind.

Durch den Zusatz der vorgenannten nichtionischen Tenside der allgemeinen Formel R¹-CH(OH)CH₂O-(AO)_{w}-(A'O)ₓ-(A"O)_{y}-(A"'O)_{z}-R², nachfolgend auch als "Hydroxymischether" bezeichnet, kann die Reinigungsleistug erfindungsgemäßer Enzym-haltiger Zubereitungen deutlich verbessert werden und zwar sowohl im Vergleich zu Tensid-freien Systemen wie auch im Vergleich zu Systemen, die alternative nichtionischen Tenside, beispielsweise aus der Gruppe der polyalkoxylierten Fettalkohole enthalten.

Durch den Einsatz dieser nichtionischen Tenside mit einer oder mehreren freien Hydroxylgruppe an einem oder beiden endständigen Alkylreste kann die Stabilität der in den erfindungsgemäßen Wasch- oder Reinigungsmittelzubereitungen enthaltenen Enzyme deutlich verbessert werden.

Bevorzugt werden insbesondere solche endgruppenverschlossene poly(oxyalkylierten) Niotenside, die, gemäß der Formel R'O[CH₂CH₂O]ₓCH₂CH(OH)R², neben einem Rest R¹, welcher für lineare oder verzweigte, gesättigte oder ungesättigte, aliphatische oder aromatische Kohlenwasserstoffreste mit 2 bis 30 Kohlenstoffatomen, vorzugsweise mit 4 bis 22 Kohlenstoffatomen steht, weiterhin einen linearen oder verzweigten, gesättigten oder ungesättigten, aliphatischen oder aromatischen Kohlenwasserstoffrest R² mit 1 bis 30 Kohlenstoffatomen aufweisen, wobei x für Werte zwischen 1 und 90, vorzugsweise für Werte zwischen 30 und 80 und insbesondere für Werte zwischen 30 und 60 steht.

Besonders bevorzugt sind Tenside der Formel R¹O[CH₂CH(CH₃)O]ₓ[CH₂CH₂O]_{y}CH₂CH(OH)R², in der R¹ für einen linearen oder verzweigten aliphatischen Kohlenwasserstoffrest mit 4 bis 18 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 1,5 sowie y für einen Wert von mindestens 15 steht.

Zur Gruppe dieser nichtionischen Tenside zählen beispielsweise die C₂₋₂₆ Fettatkohol-(PO)₁-(EO)_{15 40}-2-hydroxyalkylether, insbesondere auch die C₈₋₁₀ Fettalkohol-(PO)₁-(EO)₂₂-2-hydroxydecylether.

Besonders bevorzugt werden weiterhin solche endgruppenverschlossene poly(oxyalkylierten) Niotenside der Formel R¹O[CH₂CH₂O]ₓ[CH₂CH(R³)O]_{y}CH₂CH(OH)R², in der R¹ und R² unabhängig voneinander für einen linearen oder verzweigten, gesättigten oder ein- bzw. mehrfach ungesättigten Kohlenwasserstoffrest mit 2 bis 26 Kohlenstoffatomen steht, R³ unabhängig voneinander ausgewählt ist aus -CH₃, -CH₂CH₃, -CH₂CH₂-CH₃, -CH(CH₃)₂, vorzugsweise jedoch für -CH₃ steht, und x und y unabhängig voneinander für Werte zwischen 1 und 32 stehen, wobei Niotenside mit R³ = -CH₃ und Werten für x von 15 bis 32 und y von 0,5 und 1,5 ganz besonders bevorzugt sind.

Weitere bevorzugt einsetzbare Niotenside sind die endgruppenverschlossenen poly(oxyalkylierten) Niotenside der Formel R¹O[CH₂CH(R³)O]ₓ[CH₂]ₖCH(OH)[CH₂]ⱼOR², in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der oben stehenden Formel R¹O[CH₂CH(R³)O]ₓ[CH₂]ₖCH(OH)[CH₂]ⱼOR² unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der oben stehenden Formel unterschiedlich sein, falls x ≥ 2 ist. Hierdurch kann die Alkylenoxideinheit in der eckigen Klammer variiert werden. Steht x beispielsweise für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt.

Besonders bevorzugte endgruppenverschlossene poly(oxyalkylierte) Alkohole der oben stehenden Formel weisen Werte von k = 1 und j = 1 auf, so dass sich die vorstehende Formel zu R'O[CH₂CH(R')O]ₓCH₂CH(OH)CH₂OR² vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt.

Als besonders wirkungsvoll haben sich schließlich die nichtionischen Tenside der allgemeine Formel R¹-CH(OH)CH₂O-(AO)_{w}-R² erwiesen, in der
- R¹ für einen geradkettigen oder verzweigten, gesättigten oder ein- bzw. mehrfach ungesättigten C₆₋₂₄-Alkyl- oder -Alkenylrest steht;
- R² für einen linearen oder verzweigten Kohlenwasserstoffrest mit 2 bis 26 Kohlenstoffatomen steht;
- A für einen Rest aus der Gruppe CH₂CH₂, -CH₂CH₂-CH₂, -CH₂-CH(CH₃) steht, und
- w für Werte zwischen 1 und 120, vorzugsweise 10 bis 80, insbesondere 20 bis 40 steht

Zur Gruppe dieser nichtionischen Tenside zählen beispielsweise die C₄₋₂₂ Fettalkohol-(EO)₁₀₋₈₀-2-hydroxyalkylether, insbesondere auch die C₈₋₁₂ Fettalkohol-(EO)₂₂-2-hydroxydecylether und die C₄-₂₂ Fettalkohol-(EO)₄₀₋₈₀-2-hydroxyalkylether

Bevorzugte Wasch- oder Reinigungsmittel sind dadurch gekennzeichnet, dass das Wasch- oder Reinigungsmittel mindestens ein nichtionisches Tensid, vorzugsweise ein nichtionisches Tensid aus der Gruppe der Hydroxymischether, enthält, wobei der Gewichtsanteil des nichtionischen Tensids am Gesamtgewicht des Wasch- oder Reinigungsmittels vorzugsweise 0,2 bis 10 Gew.-%, bevorzugt 0,4 bis 7,0 Gew.-% und insbesondere 0,6 bis 6,0 Gew.-% beträgt.

Bevorzugte erfindungsgemäße Wasch- oder Reinigungsmittel zur Verwendung in maschinellen Geschirrspülverfahren enthalten neben den zuvor beschriebenen nichtionischen Tensiden weitere Tenside, insbesondere amphotere Tenside enthalten. Der Anteil anionischer Tenside am Gesamtgewicht dieser Wasch- oder Reinigungsmittel ist jedoch vorzugsweise begrenzt. So sind bevorzugte maschinelle Geschirrspülmittel dadurch gekennzeichnet, dass diese bezogen auf ihr Gesamtgewicht weniger als 5, 0 Gew.-%, vorzugsweise weniger als 3,0 Gew.-%, besonders bevorzugt weniger als 2,0 Gew.-% Aniontensid enthalten. Auf den Einsatz von anionischen Tensiden in größerer Menge wird dabei insbesondere zur Vermeidung einer übermäßigen Schaumentwicklung verzichtet.

Ein weiterer bevorzugter Bestandteil erfindungsgemäßer Wasch- oder Reinigungsmittel sind Komplexbildner. Besonders bevorzugte Komplexbildner sind die Phosphonate. Die komplexbildenden Phosphonate umfassen neben der 1-Hydroxyethan-1,1-diphosphonsäure eine Reihe unterschiedlicher Verbindungen wie beispielsweise Diethylentriaminpenta(methylenphosphonsäure) (DTPMP). In dieser Anmeldung bevorzugt sind insbesondere Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Ein im Rahmen dieser Anmeldung bevorzugtes Wasch- oder Reinigungsmittel enthält ein oder mehrere Phosphonat(e) aus der Gruppe
a) Aminotrimethylenphosphonsäure (ATMP) und/oder deren Salze;
b) Ethylendiamintetra(methylenphosphonsäure) (EDTMP) und/oder deren Salze;
c) Diethylentriaminpenta(methylenphosphonsäure) (DTPMP) und/oder deren Salze;
d) 1-Hydroxyethan-1,1-diphosphonsäure (HEDP) und/oder deren Salze;
e) 2-Phosphonobutan-1,2,4-tricarbonsäure (PBTC) und/oder deren Salze;
f) Hexamethylendiamintetra(methylenphosphonsäure) (HDTMP) und/oder deren Salze;
g) Nitrilotri(methylenphosphonsäure) (NTMP) und/oder deren Salze.

Besonders bevorzugt werden Wasch- oder Reinigungsmittel, welche als Phosphonate 1-Hydroxyethan-1,1-diphosphonsäure (HEDP) oder Diethylentriaminpenta(methylenphosphonsäure) (DTPMP) enthalten. Selbstverständlich können die erfindungsgemäßen Wasch- oder Reinigungsmittel zwei oder mehr unterschiedliche Phosphonate enthalten. Bevorzugte erfindungsgemäße Wasch- oder Reinigungsmittel sind dadurch gekennzeichnet, dass das Wasch-oder Reinigungsmittel mindestens einen Komplexbildner aus der Gruppe der Phosphonate, vorzugsweise 1-Hydroxyethan-1,1-diphosphonat, enthält, wobei der Gewichtsanteil des Phosphonat am Gesamtgewicht des Wasch- oder Reinigungsmittels vorzugsweise 0,1 und 8,0 Gew.-%, bevorzugt 0,2 und 5,0 Gew.-% und insbesondere 0,5 und 3,0 Gew.-% beträgt.

Die erfindungsgemäßen Wasch- oder Reinigungsmittel enthalten weiterhin vorzugsweise Gerüststoff. Zu den Gerüststoffe zählen dabei insbesondere die Silikate, Carbonate, organische Cobuilder und -wo keine ökologischen Vorurteile gegen ihren Einsatz bestehen- auch die Phosphate.

Organische Cobuildersubstanzen können gewünschtenfalls in Mengen bis zu 40 Gew.-%, insbesondere bis zu 25 Gew.-% und vorzugsweise von 1 Gew.-% bis 8 Gew.-% enthalten sein.

Unter der Vielzahl der kommerziell erhältlichen Phosphate haben die Alkalimetallphosphate unter besonderer Bevorzugung von Pentanatriumtriphosphat, Na₅P₃O₁₀ (Natriumtripolyphosphat) bzw. Pentakaliumtriphosphat, K₅P₃O₁₀ (Kaliumtripolyphosphat) für die erfindungsgemäßen Mittel die größte Bedeutung. Werden im Rahmen der vorliegenden Anmeldung Phosphate als reinigungsaktive Substanzen in dem Wasch- oder Reinigungsmittel eingesetzt, so enthalten bevorzugte Mittel diese(s) Phosphat(e), vorzugsweise Pentakaliumtriphosphat, wobei der Gewichtsanteil des Phosphats am Gesamtgewicht des Wasch- oder Reinigungsmittels vorzugsweise 5,0 und 40 Gew.-%, bevorzugt 10 und 30 Gew.-% und insbesondere 12 und 25 Gew.-% beträgt.

Als organische Cobuilder sind insbesondere Polycarboxylate / Polycarbonsäuren, polymere Polycarboxylate, Asparaginsäure, Polyacetale, Dextrine, weitere organische Cobuilder sowie Phosphonate zu nennen. Diese Stoffklassen werden nachfolgend beschrieben.

Brauchbare organische Gerüstsubstanzen sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen. Mit besonderem Vorzug wird als Gerüstsubstanz die Citronensäure oder Salze der Citronensäure eingesetzt. Weitere besonders bevorzugte Gerüstsubstanzen sind ausgewählt unter Methylglycindiessidsäure (MGDA), Glutaminsäurediacetat (GLDA), Asparaginsäurediacetat (ASDA), Hydroxyethyliminodiacetat (HEIDA), Iminodisuccinat (IDS) und Ethylendiamindisuccinat (EDDS), Carboxymethylinulin und Polyaspartat.

Als Gerüststoffe sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 70000 g/mol, vorzugsweise 20000 bis 50000 g/mol und insbesondere 30000 bis 40000 g/mol.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate.

Zur Verbesserung der Reinigungsleistung und/oder zur Einstellung der Viskosität enthalten bevorzugte Wasch- oder Reinigungsmittel mindestens ein hydrophob modifiziertes Polymer, vorzugsweise ein hydrophob modifziertes Carbonsäuregruppen-haltiges Polymer, wobei der Gewichtsanteil des hydrophob modifizierten Polymers am Gesamtgewicht des Wasch- oder Reinigungsmittels vorzugsweise 0,1 bis 10 Gew.-%, bevorzugt zwischen 0,2 und 8,0 Gew.-% und insbesondere 0,4 bis 6,0 Gew.-% beträgt.

In Ergänzung zu den zuvor beschriebenen Gerüststoffen können in dem Wasch- oder Reinigungsmittel reinigungsaktive Polymere enthalten sein. Der Gewichtsanteil der reinigungsaktiven Polymere am Gesamtgewicht erfindungsgemäßer maschineller Wasch- oder Reinigungsmittel beträgt vorzugsweise 0,1 bis 20 Gew.-%, vorzugsweise 1,0 bis 15 Gew.-% und insbesondere 2,0 bis 12 Gew.-%.

Als reinigungsaktive Polymere werden vorzugsweise Sulfonsäuregruppen-haltige Polymere, insbesondere aus der Gruppe der copolymeren Polysulfonate, eingesetzt. Diese copolymeren Polysulfonate enthalten neben Sulfonsäuregruppen-haltigem(n) Monomer(en) wenigstens ein Monomer aus der Gruppe der ungesättigten Carbonsäuren.

Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist.

Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen. Einsetzbar sind selbstverständlich auch die ungesättigten Dicarbonsäuren.

Bei den Sulfonsäuregruppen-haltigen Monomeren sind solche der Formel R⁵(R⁶)C=C(R⁷)-X-SO₃H bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste oder für - COOH oder -COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, -C(O)-NH-C(CH₃)₂-CH₂- und -C(O)-NH-CH(CH₂CH₃)-.

Unter diesen Monomeren bevorzugt sind solche der Formeln H₂C=CH-X-SO₃H, H₂C=C(CH₃)-X-SO₃H und HO₃S-X-(R⁶)C=C(R⁷)-X-SO₃H, in denen R⁶ und R⁷ unabhängig voneinander ausgewählt sind aus -H, -CH₃, -CH₂CH₃, -CH₂CH₂CH₃, -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-,-C(O)-NH-C(CH₃)₂-CH₂- und -C(O)-NH-CH(CH₂CH₃)-.

Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze.

In den Polymeren können die Sulfonsäuregruppen ganz oder teilweise in neutralisierter Form vorliegen. Der Einsatz von teil- oder vollneutralisierten sulfonsäuregruppenhaltigen Copolymeren ist erfindungsgemäß bevorzugt.

Die Molmasse der erfindungsgemäß bevorzugt eingesetzten Sulfo-Copolymere kann variiert werden, um die Eigenschaften der Polymere dem gewünschten Verwendungszweck anzupassen. Bevorzugte maschinelle Geschirrspülmittel sind dadurch gekennzeichnet, dass die Copolymere Molmassen von 2000 bis 200.000 gmol⁻¹, vorzugsweise von 4000 bis 25.000 gmol⁻¹ und insbesondere von 5000 bis 15.000 gmol⁻¹ aufweisen.

In einer weiteren bevorzugten Ausführungsform umfassen die Copolymere neben Carboxylgruppen-haltigem Monomer und Sulfonsäuregruppen-haltigen Monomer weiterhin wenigstens ein nichtionisches, vorzugsweise hydrophobes Monomer. Durch den Einsatz dieser hydrophob modifizierten Polymere konnte insbesondere die Klarspülleistung erfindungsgemäßer maschineller Geschirrspülmittel verbessert werden.

Wasch- oder Reinigungsmittel, enthaltend ein Copolymer, umfassend
i) Carbonsäuregruppen-haltige Monomer(e)
ii) Sulfonsäuregruppen-haltige Monomer(e)
iii) nichtionische Monomer(e).
werden erfindungsgemäß bevorzugt. Durch den Einsatz dieser Terpolymere konnte die Klarspülleistung erfindungsgemäßer maschineller Geschirrspülmittel gegenüber vergleichbaren Geschirrspülmitteln, die Sulfopolymere ohne Zusatz nichtionischer Monomere enthalten, verbessert werden.

Als nichtionische Monomere werden vorzugsweise Monomere der allgemeinen Formel R¹(R²)C=C(R³)-X-R⁴ eingesetzt, in der R¹ bis R³ unabhängig voneinander für -H, -CH₃ oder -C₂H₅ steht, X für eine optional vorhandene Spacergruppe steht, die ausgewählt ist aus -CH₂-, -C(O)O- und -C(O)-NH-, und R⁴ für einen geradkettigen oder verzweigten gesättigten Alkylrest mit 2 bis 22 Kohlenstoffatomen oder für einen ungesättigten, vorzugsweise aromatischen Rest mit 6 bis 22 Kohlenstoffatomen steht.

Besonders bevorzugte nichtionische Monomere sind Buten, Isobuten, Penten, 3-Methylbuten, 2-Methylbuten, Cyclopenten, Hexen, Hexen-1, 2-Methlypenten-1, 3-Methlypenten-1, Cyclohexen, Methylcyclopenten, Cyclohepten, Methylcyclohexen, 2,4,4-Trimethylpenten-1, 2,4,4-Trimethylpenten-2, 2,3-Dimethylhexen-1, 2,4-Diemthylhexen-1, 2,5-Dimethlyhexen-1, 3,5-Dimethylhexen-1, 4,4-Dimehtylhexan-1, Ethylcyclohexyn, 1-Octen, α-Olefine mit 10 oder mehr Kohlenstoffatomen wie beispielsweise 1-Decen, 1-Dodecen, 1-Hexadecen, 1-Oktadecen und C22-α-Olefin, 2-Styrol, α-Methylstyrol, 3-Methylstyrol, 4-Propylstryol, 4-Cyclohexylstyrol, 4-Dodecylstyrol, 2-Ethyl-4-Benzylstyrol, 1-Vinylnaphthalin, 2,Vinylnaphthalin, Acrylsäuremethylester, Acrylsäureethylester, Acrylsäurepropylester, Acrylsäurebutylester, Acrylsäurepentylester, Acrylsäurehexylester, Methacrylsäuremethylester, *N*-(Methyl)acrylamid, Acrylsäure-2-Ethylhexylester, Methacrylsäure-2-Ethylhexylester, *N*-(2-Ethylhexyl)acrylamid, Acrylsäureoctylester, Methacrylsäureoctylester, *N*-(Octyl)acrylamid, Acrylsäurelaurylester, Methacrylsäurelaurylester, *N*-(Lauryl)acrylamid, Acrylsäurestearylester, Methacrylsäurestearylester, *N*-(Stearyl)acrylamid, Acrylsäurebehenylester, Methacrylsäurebehenylester und *N*-(Behenyl)acrylamid oder deren Mischungen.

Der Gewichtsanteil der Sulfonsäuregruppen-haltigen Copolymere am Gesamtgewicht erfindungsgemäßer Wasch- oder Reinigungsmittel beträgt vorzugsweise 0,1 bis 15 Gew.-%, vorzugsweise 1,0 bis 12 Gew.-% und insbesondere 2,0 bis 10 Gew.-%.

Die erfindungsgemäßen Wasch- oder Reinigungsmittel können in den dem Fachmann bekannten Konfektionsformen, also beispielsweise in fester oder flüssiger Form aber auch als Kombination fester und flüssiger Angebotsformen vorliegen. Als feste Angebotsformen eignen sich insbesondere Pulver, Granulate, Extrudate oder Kompaktate, insbesondere Tabletten.

Ein Stoff, z.B. eine Zusammensetzung oder ein Mittel ist gemäß Definition der Erfindung festförmig, wenn sie bei 25 °C und 1013 mbar im festen Aggregatzustand vorliegt.

Ein Stoff, z.B. eine Zusammensetzung oder ein Mittel ist gemäß Definition der Erfindung flüssig, wenn sie bei 25 °C und 1013 mbar im flüssigen Aggregatzustand vorliegt. Dabei umfasst flüssig auch gelförmig.

Feste, insbesondere pulverförmige, erfindungsgemäße Wasch- oder Reinigungsmittel, können neben den erfindungsgemäßen Enzymen im Prinzip alle bekannten und in derartigen Mitteln üblichen Inhaltsstoffe enthalten, wobei mindestens ein weiterer Inhaltsstoff in dem Mittel vorhanden ist. Die erfindungsgemäßen Mittel können insbesondere Buildersubstanzen, oberflächenaktive Tenside, Bleichmittel auf Basis organischer und/oder anorganischer Persauerstoffverbindungen, Bleichaktivatoren, wassermischbare organische Lösungsmittel, Enzyme, Sequestrierungsmittel, Elektrolyte, pH-Regulatoren und weitere Hilfsstoffe wie optische Aufheller, Vergrauungsinhibitoren, Schaumregulatoren sowie Farb- und Duftstoffe sowie Kombinationen hiervon enthalten.

In einer Ausführungsform umfasst ein Wasch- oder Reinigungsmittel, ferner
- 5 Gew.-% bis 70 Gew.-%, insbesondere 5 Gew.-% bis 30 Gew.-% Tenside und/oder
- 10 Gew.-% bis 65 Gew.-%, insbesondere 12 Gew.-% bis 60 Gew. -% wasserlösliches oder wasserdispergierbares anorganisches Buildermaterial und/oder
- 0,5 Gew.-% bis 10 Gew.-%, insbesondere 1 Gew.-% bis 8 Gew.-%, wasserlösliche organische Buildersubstanzen und/oder
- 0,01 bis 15 Gew.-% feste anorganische und/oder organische Säuren beziehungsweise saure Salze und/oder
- 0,01 bis 5 Gew.-% Komplexbildner für Schwermetalle und/oder
- 0,01 bis 5 Gew.-% Vergrauungsinhibitor und/oder
- 0,01 bis 5 Gew. -% Farbübertragungsinhibitor und/oder
- 0,01 bis 5 Gew.-% Schauminhibitor.

Optional kann das Mittel ferner optische Aufheller umfassen, bevorzugt von 0,01 bis 5 Gew-%.

Die Herstellung erfindungsgemäßer fester Mittel bietet keine Schwierigkeiten und kann auf bekannte Weise, zum Beispiel durch Sprühtrocknen oder Granulation, erfolgen, wobei Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein einen Extrusionsschritt aufweisendes Verfahren bevorzugt.

Zur Herstellung von erfindungsgemäßen festen Mitteln in Tablettenform, die einphasig oder mehrphasig, einfarbig oder mehrfarbig und insbesondere aus einer Schicht oder aus mehreren, insbesondere aus zwei Schichten bestehen können, geht man vorzugsweise derart vor, dass man alle Bestandteile - gegebenenfalls je einer Schicht - in einem Mischer miteinander vermischt und das Gemisch mittels herkömmlicher Tablettenpressen, beispielsweise Exzenterpressen oder Rundläuferpressen, mit Presskräften im Bereich von etwa 50 bis 100 kN, vorzugsweise bei 60 bis 70 kN verpresst. Insbesondere bei mehrschichtigen Tabletten kann es von Vorteil sein, wenn mindestens eine Schicht vorverpresst wird. Dies wird vorzugsweise bei Presskräften zwischen 5 und 20 kN, insbesondere bei 10 bis 15 kN durchgeführt. Man erhält so problemlos bruchfeste und dennoch unter Anwendungsbedingungen ausreichend schnell lösliche Tabletten mit Bruch- und Biegefestigkeiten von normalerweise 100 bis 200 N, bevorzugt jedoch über 150 N. Vorzugsweise weist eine derart hergestellte Tablette ein Gewicht von 10 g bis 50 g, insbesondere von 15 g bis 40 g auf. Die Raumform der Tabletten ist beliebig und kann rund, oval oder eckig sein, wobei auch Zwischenformen möglich sind. Ecken und Kanten sind vorteilhafterweise abgerundet. Runde Tabletten weisen vorzugsweise einen Durchmesser von 30 mm bis 40 mm auf. Insbesondere die Größe von eckig oder quaderförmig gestalteten Tabletten, welche überwiegend über die Dosiervorrichtung beispielsweise der Geschirrspülmaschine eingebracht werden, ist abhängig von der Geometrie und dem Volumen dieser Dosiervorrichtung. Beispielhaft bevorzugte Ausführungsformen weisen eine Grundfläche von (20 bis 30 mm) x (34 bis 40 mm), insbesondere von 26x36 mm oder von 24x38 mm auf.

Ein erfindungsgemäßes festes Mittel enthält vorzugsweise mindestens einen wasserlöslichen und/oder wasserunlöslichen, organischen und/oder anorganischen Builder. Zu den wasserlöslichen organischen Buildersubstanzen gehören die oben genannten organischen Gerüstsubstanzen.

Als wasserlösliche anorganische Buildermaterialien kommen für feste Mittel insbesondere Alkalisilikate, Alkalicarbonate und Alkaliphosphate, die in Form ihrer alkalischen, neutralen oder sauren Natrium- oder Kaliumsalze vorliegen können, in Betracht. Beispiele hierfür sind in dieser Anmeldung weiter oben genannt.

Als wasserdispergierbare anorganische Buildermaterialien für feste Mittel werden insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt. Unter diesen sind die kristallinen Natriumalumosilikate in Waschmittelqualität, insbesondere Zeolith A, P und gegebenenfalls X, allein oder in Mischungen, beispielsweise in Form eines Co-Kristallisats aus den Zeolithen A und X (Vegobond® AX, ein Handelsprodukt der Condea Augusta S.p.A.), bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das nach den Angaben der deutschen Patentschrift DE 24 12 837 bestimmt werden kann, liegt in der Regel im Bereich von 100 bis 200 mg CaO pro Gramm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 22, insbesondere 1,9 bis 4 und y eine Zahl von 0 bis 33 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl β- als auch δ-Natriumdisilikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäßer Mittel eingesetzt. Kristalline schichtförmige Silikate der oben angegebenen Formel (I) werden von der Fa. Clariant GmbH unter dem Handelsnamen Na-SKS vertrieben, z.B. Na-SKS-1 (Na₂Si₂₂O₄₅·H₂O, Kenyait), Na-SKS-2 (Na₂Si₁₄O₂₉·xH₂O, Magadiit), Na-SKS-3 (Na₂Si₈O₁₇·xH₂O) oder Na-SKS-4 (Na₂Si₄O₉·xH₂O, Makatit). Von diesen eignen sich vor allem Na-SKS-5 (α-Na₂Si₂O₅), Na-SKS-7 (β-Na₂Si₂O₅, Natrosilit), Na-SKS-9 (NaHSi₂O₅·3H₂O), Na-SKS-10 (NaHSi₂O₅·3H₂O, Kanemit), Na-SKS-11 (t-Na₂Si₂O₅) und Na-SKS-13 (NaHSi₂O₅), insbesondere aber Na-SKS-6 (δ-Na₂Si₂O₅). In einer bevorzugten Ausgestaltung erfindungsgemäßer Mittel setzt man ein granulares Compound aus kristallinem Schichtsilikat und Citrat, aus kristallinem Schichtsilikat und oben genannter (co-)polymerer Polycarbonsäure, oder aus Alkalisilikat und Alkalicarbonat ein, wie es beispielsweise unter dem Namen Nabion® 15 im Handel erhältlich ist.

Buildersubstanzen sind in den erfindungsgemäßen festen Mitteln vorzugsweise in Mengen bis zu 75 Gew.-%, insbesondere 5 Gew.-% bis 50 enthalten.

Als für den Einsatz in erfindungsgemäßen festen Mitteln geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Waschbedingungen Wasserstoffperoxid abgebende anorganische Salze, zu denen Perborat, Percarbonat, Persilikat und/oder Persulfat wie Caroat gehören, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Falls ein erfindungsgemäßes Mittel Persauerstoffverbindungen enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat kann zweckdienlich sein.

Als Bleichaktivatoren können in den festen Mitteln Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren beschriebene Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam. Die hydrophil substituierten Acylacetale und die Acyllactame werden ebenfalls bevorzugt eingesetzt. Auch Kombinationen konventioneller Bleichaktivatoren können eingesetzt werden. Derartige Bleichaktivatoren können, insbesondere bei Anwesenheit obengenannter Wasserstoffperoxid-liefernder Bleichmittel, im üblichen Mengenbereich, vorzugsweise in Mengen von 0,5 Gew.-% bis 10 Gew.-%, insbesondere 1 Gew.-% bis 8 Gew.-%, bezogen auf gesamtes Mittel, enthalten sein, fehlen bei Einsatz von Percarbonsäure als alleinigem Bleichmittel jedoch vorzugsweise ganz.

Zusätzlich zu den konventionellen Bleichaktivatoren oder an deren Stelle können in festen Mitteln auch Sulfonimine und/oder bleichverstärkende Übergangsmetallsalze beziehungsweise Übergangsmetallkomplexe als sogenannte Bleichkatalysatoren enthalten sein.

Zu den erfindungsgemäßen festen Darreichungsformen zählen Extrudate, Granulate, Tabletten oder Pouches enthaltend feste Mittel, die sowohl in Großgebinden als auch portionsweise abgepackt vorliegen können. Alternativ liegt das Mittel als rieselfähiges Pulver vor, insbesondere mit einem Schüttgewicht von 300 g/l bis 1200 g/l, insbesondere 500 g/l bis 900 g/l oder 600 g/l bis 850 g/l.

Ausführungsformen der vorliegenden Erfindung umfassen alle festen, pulverförmigen, flüssigen, gelförmigen oder pastösen Darreichungsformen der Mittel, die gegebenenfalls auch aus mehreren Phasen bestehen können sowie in komprimierter oder nicht komprimierter Form vorliegen können. Eine weitere Ausführungsform der Erfindung stellen daher Mittel dar, die dadurch gekennzeichnet sind, dass sie als Einkomponentensystem vorliegen. Solche Mittel bestehen bevorzugt aus einer Phase. Selbstverständlich können erfindungsgemäße Mittel aber auch aus mehreren Phasen bestehen, zum Beispiel zwei flüssige, zwei feste oder eine flüssige und eine feste Phase. In einer weiteren Ausführungsform der Erfindung ist das Wasch- oder Reinigungsmittel daher dadurch gekennzeichnet sind, dass es in mehrere Komponenten aufgeteilt ist.

Die flüssigen Angebotsformen auf Basis von Wasser und/oder organischen Lösungsmitteln können verdickt, in Form von Gelen vorliegen.

Die erfindungsgemäßen Wasch- oder Reinigungsmittel liegen vorzugsweise in flüssiger Form vor. Bevorzugte Wasch- oder Reinigungsmittel enthalten bezogen auf ihr Gesamtgewicht mehr als 40 Gew.-%, vorzugsweise zwischen 50 und 90 Gew.-% und insbesondere zwischen 60 und 80 Gew.-% Wasser.

Als einen weiteren Bestandteil können die erfindungsgemäßen Wasch- oder Reinigungsmittel ein organisches Lösungsmittel enthalten. Der Zusatz organischer Lösungsmittel wirkt sich vorteilhaft auf die Enzymstabilität und die Reinigungsleistung dieser Mittel aus. Bevorzugte organische Lösungsmittel stammen aus der Gruppe ein- oder mehrwertigen Alkohole, Alkanolamine oder Glykolether. Vorzugsweise werden die Lösungsmittel ausgewählt aus Ethanol, n- oder i-Propanol, Butanol, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Etheylenglykolmono-n-butylether, Diethylenglykolmethylether, Di-ethylenglykolethylether, Propylenglykolmethyl-, -ethyl- oder -propylether, Dipropylenglykolmethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether sowie Mischungen dieser Lösungsmittel. Der Gewichtsanteil dieser organischen Lösungsmittel am Gesamtgewicht erfindungsgemäßer Wasch- oder Reinigungsmittel beträgt vorzugsweise 0,1 bis 10 Gew.-%, bevorzugt 0,2 bis 8,0 Gew.-% und insbesondere 0,5 bis 5,0 Gew.-%. Ein besonders bevorzugtes und in Bezug auf die Stabilisierung der Wasch- oder Reinigungsmittel besonders wirksames organisches Lösungsmittel ist das Glycerin sowie das 1,2 Propylenglykol. Flüssige Wasch- oder Reinigungsmittel die mindestens ein Polyol, vorzugsweise aus der Gruppe Glycerin und 1,2-Propylenglycol enthalten, wobei der Gewichtsanteil des Polyols am Gesamtgewicht des Wasch- oder Reinigungsmittels vorzugsweise 0,1 und 10 Gew.-%, bevorzugt 0,2 und 8,0 Gew.-% und insbesondere 0,5 und 5,0 Gew.-% beträgt, werden erfindungsgemäß bevorzugt.

Weitere bevorzugte organische Lösungsmittel sind die organischen Amine und Alkanolamine. Die erfindungsgemäßen Wasch- oder Reinigungsmittel enthalten diese Amine vorzugsweise in Mengen von 0,1 bis 10 Gew.-%, bevorzugt von 0,2 bis 8,0 Gew.-% und insbesondere von 0,5 bis 5,0 Gew.-%, jeweils bezogen auf ihr Gesamtgewicht. Ein besonders bevorzugtes Alkanolamin ist das Ethanolamin.

Ein weiterer bevorzugter Bestandteil der erfindungsgemäßen Wasch- oder Reinigungsmittel ist ein Zuckeralkohol (Alditol). Die Gruppe der Alditole umfasst nichtcyclische Polyole der Formel HOCH₂[CH(OH)]ₙCH₂OH. Zu den Alditolen zählen beispielsweisee Mannit (Mannitol), Isomalt, Lactit, Sorbit (Sorbitol) und Xylit (Xylitol), Threit, Erythrit und Arabit. Als in Bezug auf die Enzymstabilität besonders vorteilhaft hat sich das Sorbitol erwiesen. Der Gewichtsanteil des Zuckeralkohols am Gesamtgewicht des Wasch- oder Reinigungsmittels beträgt vorzugsweise 1,0 bis 10 Gew.-%, bevorzugt 2,0 bis 8,0 Gew.-% und insbesondere 3,0 bis 6,0 Gew.-%.

Erfindungsgemäße flüssige Wasch- oder Reinigungsmittel werden vorzugsweise in mehrphasiger Form, das heißt durch Kombination von zwei oder mehr voneinander getrennten unterschiedlichen flüssigen Wasch- oder Reinigungsmitteln konfektioniert. Diese Art der Konfektionierung erhöht die Stabilität des Wasch- oder Reinigungsmittels und verbessert dessen Reinigungsleistung. Ein erfindungsgemäß bevorzugtes Wasch- oder Reinigungsmittel ist dadurch gekennzeichnet, dass es ein Verpackungsmittel und zwei in diesem Verpackungsmittel befindlichen voneinander getrennte flüssige Wasch- oder Reinigungsmittel A und B umfasst, wobei die Zusammensetzung A
a) mindestens eine erfindungsgemäße modifizierte Protease;
b) mindestens ein weiteres, von der erfindungsgemäßen Protease verschiedenes Enzym,
c) 10 bis 84,9 Gew.-% Gerüststoff(e);
d) 15 bis 89,9 Gew.-% Wasser; enthält und
   die Zusammensetzung B
e) 10 bis 75 Gew.% Gerüststoff(e);
f) 25 bis 90 Gew.-% Wasser;
   enthält.

Ein weiterer Erfindungsgegenstand ist ein Verfahren zur Reinigung von Textilien oder harten Oberflächen, das dadurch gekennzeichnet ist, dass in mindestens einem Verfahrensschritt ein erfindungsgemäßes Mittel angewendet wird.

Hierunter fallen sowohl manuelle als auch maschinelle Verfahren, wobei maschinelle Verfahren bevorzugt sind. Verfahren zur Reinigung von Textilien zeichnen sich im allgemeinen dadurch aus, daß in mehreren Verfahrensschritten verschiedene reinigungsaktive Substanzen auf das Reinigungsgut aufgebracht und nach der Einwirkzeit abgewaschen werden, oder daß das Reinigungsgut in sonstiger Weise mit einem Waschmittel oder einer Lösung oder Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels oder einer erfindungsgemäßen Kombination aus Amylase und Protease bereichert werden und stellen dann Ausführungsformen der vorliegenden Erfindung dar. Alle Sachverhalte, Gegenstände und Ausführungsformen, die für erfindungsgemäße Kombination aus Amylase und Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, daß diese Offenbarung auch für die vorstehenden erfindungsgemäßen Verfahren gilt.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Ein weiterer Erfindungsgegenstand ist die Verwendung eines erfindungsgemäßen Mittels zur Reinigung von Textilien oder von harten Oberflächen, insbesondere derart, dass die Kombination aus Amylase und Protease in einer Menge von 40µg bis 4g, vorzugsweise von 50µg bis 3g, besonders bevorzugt von 100µg bis 2g und ganz besonders bevorzugt von 200µg bis 1g eingesetzt wird.

Alle Sachverhalte, Gegenstände und Ausführungsformen, die für erfindungsgemäße Proteasen und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

### Beispiel

Das folgende Beispiel erläutert die Erfindung, ohne sie jedoch darauf einzuschränken:
Untersuchung der erfindungsgemäßen Kombinationen aus Amylase und Protease in der Waschmaschine.

Für dieses Beispiel wurden standardisiert verschmutzte Textilien eingesetzt. Es wurde die folgende Anschmutzung verwendet:
- Blut/Milch/Tusche auf Baumwolle: Produkt Nr. C-05 erhältlich von CFT (Center For Testmaterials) B.V. Vlaardingen, Niederlande

Die erfindungsgemäße Kombination aus der Protease gemäß SEQ ID NO. 4 und einer erfindungsgemäßen Amylase (Ansatz 1) sowie ein Vergleichsansatz enthaltend die Protease gemäß SEQ ID NO. 4 und Stainzyme® (Ansatz 2) wurden in der Waschmaschine vergleichend gewaschen, wobei die Waschversuche einmal direkt nach Formulierung des Waschmittels (0 Wochen) und einmal nach Lagerung des Waschmittels für 16 Wochen (16 Wochen) gemacht wurden.

Es wurde jeweils die gleiche Menge Aktivenzym eingesetzt. Die Proteasen und Amylasen wurden on top zu einem wässrigen Flüssigwaschmittel (enthaltend neben Wasser 5,5 Gew.-% 7-fach ethoxylierten C12/14-Fettakohol, 5,3 Gew.-% Natrium-C9-13-Alkylbenzolsulfonat, 4,9 Gew.-% Natrium-C12/14-Fettalkoholethersulfat mit 2 EO, 1,8 Gew.-% Zitronensäure, 3 Gew.-% C12-18-Fettsäure, 0,1 Gew.-% Diethylentriaminpenta(methylenphosphonsäure)-hepta-Natriumsalz, 1,3 Gew.-% NaOH, 3,6 Gew.-% Ethanol/Glycerin) mit pH 8,5 dosiert und bei 30°C, Wasserhärte 16°dH, in einer Miele W 1935, Gesamtwaschzeit 70 min, gewaschen.

Es wurde eine 6-fach Bestimmung durchgeführt und es wurden verschiedene Anschmutzungsmonitore gewaschen und nach der Wäsche mit einem Farbmessgerät vermessen.

Es ergaben sich folgende delta Y-Werte für den Ansatz 1 im Vergleich zu Ansatz 2:

| Lagerzeit (Wochen) | delta Y-Werte (Ansatz 1 - Ansatz 2) |
|---|---|
| 0 | 2,1 |
| 16 | 3,2 |

Man erkennt deutlich, dass die Waschleistung des Waschmittels enthaltend die Protease gemäß SEQ ID NO. 4 und die erfindungsgemäße Amylase sowohl nach 0 Wochen als auch nach 16 Wochen signifikant erhöht ist. Insbesondere zeigt sich ein größerer Effekt nach einer Lagerung von 16 Wochen, was zusätzlich auf eine Stabilisierung der Protease durch die erfindungsgemäße Amylase schließen lässt.

## Patentansprüche

1. Wasch- oder Reinigungsmittel enthaltend eine Kombination aus einer α-Amylase und einer Protease, wobei die α-Amylase **dadurch gekennzeichnet ist, dass** sie zu der in SEQ ID NO.1 angegebenen Sequenz über deren Gesamtlänge zu mindestens 89% und zunehmend bevorzugt zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% und bis zu 100% identisch ist und in der Zählung gemäß SEQ ID NO. 1 an einer oder mehreren der Positionen 180, 181, 182, 183 und 184 Deletionen aufweist, und wobei die Protease **dadurch gekennzeichnet ist, dass** sie eine Aminosäuresequenz aufweist, die zu der in SEQ ID NO. 2 angegebenen Aminosäuresequenz über deren Gesamtlänge zu mindestens 80% und zunehmend bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist, wobei die α-Amylase die Stabilität und die Leistung der Protease in dem Wasch- und Reinigungsmittel verbessert.

2. Wasch- oder Reinigungsmittel gemäß Anspruch 1, wobei die Amylase Deletionen an mindestens zwei Positionen ausgewählt aus den Positionen 180+181, 181+182, 182+183 und 183+184 in der Zählung gemäß SEQ ID NO. 1, und ganz besonders bevorzugt an den Positionen 183+184 in der Zählung gemäß SEQ ID NO. 1 aufweist.

3. Wasch- oder Reinigungsmittel gemäß Anspruch 1 und 2, wobei die Amylase die Deletionen H183* + G184* in der Zählung gemäß SEQ ID NO. 1 aufweist.

4. Wasch- oder Reinigungsmittel gemäß Anspruch 1 bis 3, wobei die Amylase in der Zählung gemäß SEQ ID NO. 1 weiterhin Aminosäuresubstitutionen an einer oder mehreren der Positionen 405, 421, 422 und 428 aufweist.

5. Wasch- oder Reinigungsmittel gemäß Anspruch 1 bis 4, wobei die Amylase in der Zählung gemäß SEQ ID NO. 1 die Substitutionen I405L; A421H, A422P und A428T aufweist.

6. Wasch- oder Reinigungsmittel gemäß Anspruch 1, wobei die Protease eine Aminosäuresequenz aufweist, die zu der in SEQ ID NO. 2 angegebenen Aminosäuresequenz über deren Gesamtlänge zu mindestens 80% und zunehmend bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist und an Position 99 in der Zählung gemäß SEQ ID NO. 2 die Aminosäure Glutaminsäure (E) aufweist.

7. Wasch- oder Reinigungsmittel gemäß Anspruch 1 bis 6, wobei die Protease eine Protease gemäß SEQ ID NO.4 ist.

8. Wasch- oder Reinigungsmittel gemäß Anspruch 1 bis 7, **dadurch gekennzeichnet, dass** die Protease in einer Menge von 0,001-0,1 Gew.-%, vorzugsweise von 0,01 bis 0,06 Gew.-% und die Amylase in einer Menge von 0,001-0,15 Gew.-%, vorzugsweise von 0,005 bis 0,012 Gew.-%, bezogen auf das Gesamtgewicht des Wasch- oder Reinigungsmittels in diesem enthalten sind.

9. Verwendung eines Wasch-oder Reinigungsmittels nach einem der Ansprüche 1-8 zum Waschen von Textilien oder Reinigen von harten Oberflächen.

10. Verfahren zur Reinigung von Textilien oder harten Oberflächen, **dadurch gekennzeichnet, dass** in mindestens einem Verfahrensschritt ein Wasch-oder Reinigungsmittel nach einem der Ansprüche 1-8 verwendet wird.

11. Verwendung einer α-Amylase zur Verbesserung der Stabilität und Leistung einer Protease in einem Wasch- und Reinigungsmittel, wobei die α-Amylase **dadurch gekennzeichnet ist, dass** sie zu der in SEQ ID NO.1 angegebenen Sequenz über deren Gesamtlänge zu mindestens 89% und zunehmend bevorzugt zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% und bis zu 100% identisch ist und in der Zählung gemäß SEQ ID NO. 1 an einer oder mehreren der Positionen 180, 181, 182, 183 und 184 Deletionen aufweist, und wobei die Protease **dadurch gekennzeichnet ist, dass** sie eine Aminosäuresequenz aufweist, die zu der in SEQ ID NO. 2 angegebenen Aminosäuresequenz über deren Gesamtlänge zu mindestens 80% und zunehmend bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist.

## Claims

1. A washing or cleaning agent containing a combination of an α-amylase and a protease, the α-amylase being **characterized in that** it is at least 89% and increasingly preferably at least 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, 99.5% and up to 100% identical to the sequence specified in SEQ ID NO.1 over the entire length thereof, and has deletions at one or more of the positions 180, 181, 182, 183 and 184 in the numbering according to SEQ ID NO.1, and the protease being **characterized in that** it has an amino acid sequence which is at least 80% and increasingly preferably at least 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5% and 99% identical to the amino acid sequence specified in SEQ ID NO. 2 over the entire length thereof, the α-amylase improving the stability and performance of the protease in the washing and cleaning agent.

2. The washing or cleaning agent according to claim 1, wherein the amylase has deletions at at least two positions selected from the positions 180 + 181, 181 + 182, 182 + 183 and 183 + 184 in the numbering according to SEQ ID NO.1, and very particularly preferably at the positions 183 + 184 in the numbering according to SEQ ID NO. 1.

3. The washing or cleaning agent according to claim 1 and 2, wherein the amylase has the deletions H183* + G184* in the numbering according to SEQ ID NO. 1.

4. The washing or cleaning agent according to claim 1 to 3, wherein the amylase also has amino acid substitutions at one or more of the positions 405, 421, 422 and 428 in the numbering according to SEQ ID NO. 1.

5. The washing or cleaning agent according to claim 1 to 4, wherein the amylase has the substitutions I405L; A421H, A422P and A428T in the numbering according to SEQ ID NO. 1.

6. The washing or cleaning agent according to claim 1, wherein the protease has an amino acid sequence which is at least 80% and increasingly preferably at least 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5% and 99% identical to the amino acid sequence specified in SEQ ID NO. 2 over the entire length thereof, and has the amino acid glutamic acid (E) at position 99 in the numbering according to SEQ ID NO. 2.

7. The washing or cleaning agent according to claim 1 to 6, wherein the protease is a protease according to SEQ ID NO.4.

8. The washing or cleaning agent according to claim 1 to 7, **characterized in that** the protease is contained in said agent in an amount of 0.001 to 0.1 wt.%, preferably of 0.01 to 0.06 wt.%, and the amylase is contained in said agent in an amount of 0.001 to 0.15 wt.%, preferably of 0.005 to 0.012 wt.%, based on the total weight of the washing or cleaning agent.

9. The use of a washing or cleaning agent according to one of claims 1 to 8 for washing textiles or cleaning hard surfaces.

10. A method for cleaning textiles or hard surfaces, **characterized in that** a washing or cleaning agent according to one of claims 1 to 8 is used in at least one method step.

11. The use of an α-amylase for improving the stability and performance of a protease in a washing and cleaning agent, the α-amylase being **characterized in that** it is at least 89% and increasingly preferably at least 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, 99.5% and up to 100% identical to the sequence specified in SEQ ID NO. 1 over the entire length thereof, and has deletions at one or more of the positions 180, 181, 182, 183 and 184 in the numbering according to SEQ ID NO.1, and the protease being **characterized in that** it has an amino acid sequence which is at least 80% and increasingly preferably at least 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5% and 99% identical to the amino acid sequence specified in SEQ ID NO. 2 over the entire length thereof.

## Revendications

1. Agent de lavage ou de nettoyage contenant une combinaison d'une α-amylase et d'une protase, dans lequel l'a-amylase est **caractérisée en ce qu'**elle est identique sur toute sa longueur à la séquence représentée dans SEQ ID NO 1 à au moins 89 % et plus préférablement à au moins 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 %, 99 %, 99,5 % et jusqu'à 100 % et présente des délétions à une ou plusieurs des positions 180, 181, 182, 183 et 184 dans le comptage selon SEQ ID NO 1, et la protéase étant **caractérisée en ce qu'**elle présente une séquence d'acides aminés qui est identique sur toute sa longueur à la séquence d'acides aminés représentée dans SEQ ID NO 2 à au moins 80 % et plus préférablement à au moins 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 % et 99 %, l'a-amylase améliorant la stabilité et la performance de la protéase dans l'agent de lavage et de nettoyage.

2. Agent de lavage ou de nettoyage selon la revendication 1, dans lequel l'amylase présente des délétions à au moins deux positions choisies parmi les positions 180 + 181, 181 + 182, 182 + 183 et 183 + 184 dans le comptage selon SEQ ID NO 1, et de manière particulièrement préférée aux positions 183 + 184 dans le comptage selon SEQ ID NO 1.

3. Agent de lavage ou de nettoyage selon les revendications 1 et 2, dans lequel l'amylase présente les délétions H183* + G184* dans le comptage selon SEQ ID NO 1.

4. Agent de lavage ou de nettoyage selon les revendications 1 à 3, dans lequel l'amylase présente en outre des substitutions d'acides aminés à une ou plusieurs des positions 405, 421, 422 et 428 dans le comptage selon SEQ ID NO 1.

5. Agent de lavage ou de nettoyage selon les revendications 1 à 4, dans lequel l'amylase présente les substitutions I405L; A421H, A422P et A428T dans le comptage selon SEQ ID NO1.

6. Agent de lavage ou de nettoyage selon la revendication 1, dans lequel la protéase présente une séquence d'acides aminés qui est identique sur toute sa longueur à la séquence d'acides aminés représentée dans SEQ ID NO 2 à au moins 80 % et plus préférablement à au moins 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95%, 95,5 %, 96 %, 96,5 %, 97 %, 97,5 %, 98 %, 98,5 % et 99 %, et présente l'acide aminé d'acide glutamique (E) à la position 99 dans le comptage selon SEQ ID NO 2.

7. Agent de lavage ou de nettoyage selon les revendications 1 à 6, dans lequel la protéase est une protéase selon SEQ ID NO 4.

8. Agent de lavage ou de nettoyage selon les revendications 1 à 7, **caractérisé en ce que** la protéase est contenue en une quantité de 0,001 à 0,1 % en poids, de préférence de 0,01 à 0,06 % en poids et l'amylase est contenue en une quantité de 0,001 à 0,15 % en poids, de préférence de 0,005 à 0,012 % en poids, sur la base du poids total de l'agent de lavage ou de nettoyage.

9. Utilisation d'un agent de lavage ou de nettoyage selon l'une des revendications 1 à 8 pour le lavage de textiles ou le nettoyage de surfaces dures.

10. Procédé pour le nettoyage de textiles ou de surfaces dures, **caractérisé en ce qu'**un agent de lavage ou de nettoyage selon l'une des revendications 1 à 8 est utilisé dans au moins une étape de procédé.

11. Utilisation d'une α-amylase pour l'amélioration de la stabilité et de la performance d'une protéase dans un agent de lavage ou de nettoyage, l'a-amylase étant **caractérisée en ce qu'**elle est identique sur toute sa longueur à la séquence représentée dans SEQ ID NO 1 à au moins 89 % et plus préférablement à au moins 90 %, 90,5 %, 91 %, 91,5 %, 92 %, 92,5 %, 93 %, 93,5 %, 94 %, 94,5 %, 95%, 95,5 %, 96 %, 96,5 %, 97 %, 97,5 %, 98 %, 98,5 %, 99 %, 99,5 % et jusqu'à 100 %, et présente des délations à une ou plusieurs des positions 180, 181, 182, 183 et 184 dans le comptage selon SEQ ID NO 1, et la protéase étant **caractérisée en ce qu'**elle présente une séquence d'acides aminés qui est identique sur toute sa longueur à la séquence d'acides aminés représentée dans SEQ ID NO 2 à au moins 80 % et plus préférablement à au moins 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95%, 95,5 %, 96 %, 96,5 %, 97 %, 97,5 %, 98 %, 98,5 % et 99 %.
